# EUROPEAN PATENT APPLICATION

(11) **EP 4 181 152 A1**
(43) Date of publication of application: **17.05.2023**
(21) Application number: 21208153.3
(22) Date of filing: 15.11.2021
(51) Int. Cl.: G16H 30/40, G16H 50/70

(54) **PROCESSING IMAGE DATA FOR ASSESSING A CLINICAL QUESTION**

(71) Applicant: Koninklijke Philips N.V., 5656 AG Eindhoven (NL)
(72) Inventor: JACOBS, Igor, 5656 AG Eindhoven (NL); PLUYTER, Jon, Ragnar, 5656 AG Eindhoven (NL); GEURTS, Lucas, Jacobus, Franciscus, 5656 AG Eindhoven (NL); RASENBERG, Diederik, 5656 AG Eindhoven (NL); VAN DER VEN, John, Cornelius, Petrus, 5656 AG Eindhoven (NL); HEESTERBEEK, Lynn, 5656 AG Eindhoven (NL); MAVROEIDIS, Dimitrios, 5656 AG Eindhoven (NL); YU, Bin, 5656 AG Eindhoven (NL)
(74) Representative: Philips Intellectual Property & Standards

(57) **Abstract**

A system (100) for processing medical image data regarding a clinical question uses patient data (110), medical image data (120) and clinical guidelines (170). The system has a processor subsystem (160) to determine, based on user input (140), a use case regarding the clinical question for a patient. The processor retrieves the patient data and image data regarding the use case to determine an anatomical object. Then, the processor selects an algorithm from an algorithm repository (AM) based on the determined anatomical object and executes the algorithm on the image data to provide the output data for assessing the clinical question. The processor may also use the codified clinical guidelines regarding the use case to select said algorithm. Effectively the most appropriate algorithm is executed for the specific use case.

## Description

### FIELD OF THE INVENTION

The invention relates to systems and computer-implemented methods for processing medical image data regarding a clinical question. The invention further relates to a computer-readable medium comprising instructions to perform one of the computer-implemented methods.

### BACKGROUND OF THE INVENTION

In oncology, early detection, timely and accurate diagnosis and staging, and subsequent selection of the appropriate treatment for each patient are critical factors to improve patient outcomes. Diagnostic imaging plays an integral role in many phases of a patient's care path. However, considerable challenges need to be overcome regarding the accuracy, efficacy, and efficiency of interpretation of radiologic imaging in oncology. Computer-aided detection and diagnosis (CAD) algorithms using artificial intelligence (AI) hold great promise in medical imaging, enabling earlier cancer detection, better cancer characterization and improved monitoring of the patient's response to treatment by supporting image analysis. However, a challenge with many algorithmic solutions is that their adoption is limited, due to a suboptimal integration in the clinical workflow and lack of a physician- and patient-centred approach.

Clinical questions may include therapy planning support regarding tumor segmentation and characterization models, organ and anatomical segmentation models, as well as advanced visualization models. The outcome of these models will be used to provide support in the planning and guidance of surgical procedures in surgical interventional planning and procedural guidance. This may be done in context of a suite of solutions, such as an abdominal / interventional suite that may include algorithmic solutions and AI applications in context of lung, liver, spleen, pancreas and kidney. Such solutions can provide guidance by applying algorithmic solutions and AI applications for decision-making on therapy options, guiding interventions and confirming treatment results. Likewise, such solutions may provide tools for more confident diagnosis and staging, treatment decisions, therapy planning and follow-up, such as support a radiologist in image reading, oncologist in interventional planning, and in general be used to support follow-up decisions.

Therefore, there is a need to improve collaboration between the physician and algorithmic and AI solutions, and achieve a holistic effect of such solutions for improving physician-algorithm collaboration on adoption and use of AI algorithms in the field, and reduce mistrust and misuse of algorithms. The problem is to develop an integrated system for assessing clinical questions like cancer detection and classification, segmentation of tumors and of critical surrounding structures that can impact oncology interventions such as vascular involvement in relation to resectability.

### SUMMARY OF THE INVENTION

It is an object of the invention to obtain a system and computer-implemented method for assessing clinical questions which makes better use of available algorithmic and AI solutions in the clinical workflow.

In accordance with a first aspect of the invention, a system is provided for processing medical image data regarding a clinical question, comprising:
- a patient data interface for accessing patient data;
- an image data interface for accessing medical image data of a patient;
- a repository interface for accessing algorithms for processing the medical image data;
- a user interface configured to receive user input from a user and to provide display data to a display to visualize output data of the system;
- a processor subsystem configured:
   to communicate with the image data interface, the patient data interface, the repository interface and the user interface;
   to determine a use case regarding the clinical question for a patient based on the user input;
      to retrieve the patient data of the patient regarding the use case;
      to determine an anatomical object based on the use case and the patient data;
      to select an algorithm from the repository based on the determined anatomical object;
      to retrieve the image data of the patient; and
      to execute the algorithm on the image data to provide the output data for assessing the clinical question.

In accordance with a further aspect of the invention, a method is provided for processing medical image data regarding a clinical question, the processing comprising:
- accessing patient data;
- accessing medical image data of a patient;
- accessing algorithms for processing the medical image data;
- receiving user input from a user;
- providing display data to visualize output data of the system;
wherein the method comprises the steps of
determining a use case regarding the clinical question for a patient based on the user input;
retrieving the patient data of the patient regarding the use case;
determining an anatomical object based on the use case and the patient data;
selecting an algorithm based on the determined anatomical object;
retrieving the image data of the patient; and
executing the algorithm on the image data to provide the output data for assessing the clinical question.

In accordance with a further aspect of the invention, a computer-readable medium is provided comprising transitory or non-transitory data representing a computer program, the computer program comprising instructions for causing a processor system to perform the above method.

The above measures effectively and automatically select appropriate algorithmic solutions for an individual patient use case, which enable automated and visual guidance for a clinical question in an image-guided intervention workflow. Upon determining a use case and the respective patient data an anatomical object is found for which a clinical question is to be assessed. The anatomical object may, for example, be an organ affected by a tumor or disease e.g. a cardiovascular use case, or a combination of one or more anatomical objects or structures that are affected by an anomaly, lesion, etc.

Advantageously, improvements are provided for patient stratification for oncological interventions and planning of such interventions, in particular by using AI algorithms from a rapidly growing amount of promising solutions to support stratification and planning for the specific patient at hand and/or using selected complex algorithm visualizations, e.g., 3D volumes with endless number of viewpoints. Said mutually reinforcing elements resolve issues with respect to mistrust and misuse of AI solutions and quality & efficiency of care in a time-pressured and high-stake interventional setting.

Optionally, in the above the system, the image data comprises 3D image data; the anatomical object is a 3D object in the 3D image data;
the selected algorithm is a slicing algorithm; and
the processor subsystem is configured
   to determine an anatomically relevant path via the 3D object;
   to execute the slicing algorithm on the image data to provide, as the display data, slices of the 3D image data that are perpendicular to the anatomically relevant path for assessing the clinical question.

Advantageously, the system uses a non-straight, anatomical relevant, path derived from a relevant anatomical structure to guide the slicing of the 3D imaging volume. A clinician is provided with a slice at the appropriate angle to optimally assess the clinical question.

Optionally, in the above the system, the selected algorithm is an analytical algorithm arranged to analyse the image data for generating a 3D anatomical model, anatomical annotations or metrics regarding the anatomical object; and the display comprises
a 2D view for displaying a guidance panel showing the anatomical annotations, metrics, codified clinical guidelines and/or a slice of the medical image data, and
a 3D view for displaying the 3D anatomical model; the processor subsystem is configured to synchronize the 3D view upon user input regarding the 2D view; or to synchronize the 2D view upon user input regarding the 3D view.

Advantageously, the system provides an integrated medical imaging workstation for diagnostic and preoperative planning support, comprising user interface elements that are synchronized across multi-dimensional viewpoints based on the use case and/or disease-specific codified guidelines. Such elements may include visualizations of patient-specific medical imaging data (CT, MRI, PET etc.), AI generated anatomical annotations, reconstructed 3D anatomical models based on AI generated annotations or guideline-driven metrics derived from AI generated annotations.

Effectively, selection of a different 3D viewpoint and view settings may be based on a selection within codified guidelines, e.g. by a user in a 2D interactive panel. A 3D viewpoint may subsequently be selected to show the most optimal view. In addition to a change of a viewpoint or model settings, a relevant slice may be selected in the slice display viewer, or a selected of a slice may be presented in an involvement panel.

Optionally, the processor subsystem may be configured to determine the use case based on detecting an anomaly in the medical image data of an area of the body of the patient; and/or based on detecting an anatomical object or structure affected by an anomaly in the medical image data of an area of the body of the patient, for enabling selecting said algorithm from the repository based on the detected anomaly and/or affected anatomical object or affected structure and retrieving the codified clinical guidelines. For example, an AI algorithm may find a tumor on a radiological image of the patient, e.g. a generic tumor detection algorithm applied to an abdominal CT scan. Subsequently, other algorithms are triggered to segment the organ in which the tumor is located, e.g. an algorithm that can segment anatomical organs, including the one in which the tumor is located. Also, an algorithm to segment the surrounding anatomical structures (e.g. the blood vessels) may be triggered, either before selection of relevant codified guidelines, or the selection of the codified guidelines is triggered based on the preceding steps for determining the use case and the anatomical object. So, the organ of interest and applicable guidelines are determined. A further algorithm may derive relevant structures or criteria from the guidelines, for example a Natural Language Processing (NLP) algorithm. Finally, anatomical structure relations and metrics may be calculated.

Optionally, the use case may be treatability based on the relation of a lesion with surrounding anatomical structures; or resectability of tumors in the vicinity of blood vessels. Advantageously, treatability may be based on the relation of the lesion with surrounding anatomical structures. The structures may be blood vessels, but also other types of anatomical structures that are of influence on the treatment. The treatment may be surgery, but may also be other treatment types that are influenced by the relation with surrounding structures: e.g. ablation, embolization, radiotherapy.

Further preferred embodiments of the system and methods according to the invention are given in the appended dependent claims, disclosure of which is incorporated herein by reference.

It will be appreciated by those skilled in the art that two or more of the above-mentioned embodiments, implementations, and/or optional aspects of the invention may be combined in any way deemed useful.

Modifications and variations of a system, computer-implemented method and/or any computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for processing medical image data regarding a clinical question;
Fig. 2 shows an example of codified guidelines;
Fig. 3 shows an example of a use case;
Fig. 4 shows system output on a display;
Fig. 5 shows an illustrative example of lung biopsy planning with 3D guidance;
Fig. 6 shows slicing of 3D volumetric data;
Fig. 7 shows an example of a tumor that is in contact with an angled blood vessel;
Fig. 8 shows an example of the imaging system having a 2D display and a 3D display;
Fig. 9 shows a computer-implemented method for processing medical image data regarding a clinical question; and
Fig. 10 shows a computer-readable medium comprising data.

It should be noted that the Figs. are purely diagrammatic and not drawn to scale. In the Figs., elements which correspond to elements already described may have the same reference numerals.

### DETAILED DESCRIPTION OF EMBODIMENTS

Patient stratification for oncological interventions and planning of such interventions can be challenging. An example is tumor resectability assessment in pancreatic cancer. Resectability depends on the extent of involvement of surrounding anatomical structures, specifically the extent of vascular involvement. This may be evaluated using radiological images (i.e. computed tomography). Clinicians face multiple challenges in the assessment. First of all, identification and localization of the tumor on radiological images can be difficult. Especially clinicians from non-expert hospitals may recognize tumor presence too late, resulting in delayed referral of patients to expert hospitals. Besides, accurate assessment of the extent of vascular involvement can be difficult, especially after neoadjuvant therapy. A variety of vascular structures and other anatomical structures need to be evaluated to get an accurate assessment of the degrees of vascular involvement, length of involvement and presence of anatomical variations (e.g. aberrant arteries) that may influence surgical strategy. For clinicians it can be a challenge to translate such evaluations that are typically done in 2D on radiological images to 3D information on patient anatomy and surgical approach.

Artificial intelligence has the potential to support and automate detection, segmentation, and characterization of the relevant anatomical structures. The amount of AI models is growing exponentially. However, selection of the AI models that are relevant for the clinical question at hand can be challenging. Besides, the output of the AI models is often not optimally integrated or presented to the clinician in a way that it effectively supports their decision-making process or oncological interventions workflows.

The system described below provides a solution by selecting the relevant algorithmic solutions and AI models based on the use case for the specific patient, for example based on codified guidelines and assessment criteria. It applies these selected algorithms to radiological images and subsequently provides in one view an overview of the relevant information derived by the algorithms in line with the information required by the guidelines. Besides, automatically relevant metrics may be derived and presented in line with the evaluation criteria.

Also, volumetric medical imaging (e.g., CT, MRI, PET, etc) may be important in the diagnosis and treatment process of complex diseases like cancerous abnormalities. Physicians are typically reviewing the patient's anatomy and potential abnormalities in standard orthogonal views/directions. These views are constructed by extracting 2D images from the volumetric dataset. These 2D images, or slices, are image planes that are perpendicular to one of the three axes of the 3D image that correspond to the anatomical cross-sections: x = coronal, y = sagittal, and z = axial. These slices are typically presented in an interface that allows the user to easily view consecutive slices and move through them quickly. These slices could then provide extensive insights into the anatomy that was imaged.

Especially in case of surgical oncology, the involved physicians must carefully examine the preoperative imaging data. This should aid them in deciding whether the patient is eligible for surgery. Main technical difficulties in performing these kinds of surgical procedures are identifying the patient-specific anatomical variation and the assessment of tumor interaction with other relevant anatomical structures and especially the vasculature that lays around the tumor. If eventually eligible for surgery, surgeons must preoperatively anticipate on medical imaging for irregular and unpredictable spatial conformation of the patient-specific anatomy and pathology.

These decisions are in current practice based on the insights extracted from the conventional medical imaging (e.g. CT, MRI, PET, etc). Surgeons need to convert these 2D imaging slices into their own mentally constructed 3D representation of the anatomy. This creates a subjective understanding of the patient anatomy and is not easily shared among the involved clinical personnel. Advancements in image processing techniques have made it possible to translate cross-sectional 2D medical imaging into three-dimensional (3D) reconstructions.

Besides evaluating the patient-specific anatomy in 3D, surgeons and radiologists must usually determine resectability of (cancerous) abnormalities by taking quantitative assessments and eventually stratify for resectability criteria based on the latest evidence based clinical guidelines. For example, in pancreatic cancer the resectability is determined based on the number of degrees circumferential contact with its surrounding vasculature.

Computer Aided Detection and Diagnosis (CAD) algorithms, a specific type of AI algorithms, can automatically annotate the tumor and all relevant anatomical structures including the vasculature. These CAD algorithms could aid physicians to detect and localize tumors, provide them with tumor characteristics (malignant y/n, cross-section, density etc) extract and quantify case specific metrics based on the AI outcomes. For example in pancreatic cancer, the tumor-vessel contact in terms of degrees and length of the tumor-vessel trajectory could be extracted from the AI findings.

Difficulties in performing these kinds of surgical procedures may be understanding the spatial conformation of the patient-specific anatomy and the assessment of tumor involvement with relevant structures. Whether a tumor is amendable for resection depends on the vascular involvement assessment. Cancer research groups and institutes usually set clear cut-off points based on involvement metrics (degrees or length of tumor contact) regarding the resectability. However, in clinical practice this quantification of tumor involvement, especially after neoadjuvant chemoradiotherapy, is experienced as highly challenging.

Clinical questions regarding neoadjuvant therapy, the right surgical technique, whether a vascular resection is needed and eligibility for surgery may be decided based on metrics like the amount of vascular involvement. If a tumor is involved with the vasculature, en bloc resection may be used. However, the incidence of vascular resections is highly dependent on the pre- and intraoperative judgement of the surgeon. This may result in tumor underdiagnosis and performing surgical procedures on patients that are not eligible for pancreaticoduodenectomy (PD). If a patient is eligible for surgery, it is crucial for surgeons and radiologists to identify potential variations in and potential stenosis of the relevant vascular anatomy to avoid the chance of complications (e.g., damaging a blood vessel) or causing potential organ ischemia (e.g., wrongly scarifying arteries).

Additionally, in comparison with a 2D display, 3D display techniques have the ability to present algorithmic segmentations of criterial anatomical structures with improved depth perception and clearer visualization of the anatomical relation between structures. However, 3D volumes can theoretically have an endless number of viewpoints. If the relevant viewpoints are not selected and presented effectively, this results in potentially lower efficiency and lack of structure in the evaluation of all available information. The described system may generate clinically relevant viewpoints based on the codified guidelines and AI-based anatomical segmentations in line with these guidelines. Also a predefined viewpoint may be enhanced by taking out irrelevant structures or make structures (partly) translucent.

When 3D volumetric imaging data, e.g., CT, PET, MRI, ultrasound, is being viewed or analysed, a user typically makes use of standard orthogonal views. These views are constructed by extracting 2D images from the 3D data. These extracted 2D images, or slices, are image planes that are perpendicular to one of the three axes of the 3D volume: x, y, z, or coronal, sagittal, axial in anatomical terms. These slices are usually presented in an interface that allows the user to easily look at consecutive slices in the same view sequentially and move through the slices quickly. The consecutive slices are extracted from adjacent points on the axis which is being viewed. These standard views provide extensive insights into the anatomy that was imaged.

A drawback of these standard views is that they don't always provide the optimal cross section for the task at hand. Take for example the clinical practice of assessing the resectability of a pancreatic tumor. For this, the clinician needs to determine the angle along which the tumor is in contact with a crucial blood vessel. Using standard slices the clinician needs to mentally reconstruct the 3D structure of the blood vessel and tumor in order to assess the situation. This can be challenging, error prone, and is not very quantitative. It is also possible for the clinician to evaluate the situation in a 3D model that was constructed using 3D segmentations of the volumetric data. Such 3D model offers a unique look at the data with a lot of flexibility. However, clinicians indicate that they typically want to verify correctness of the 3D model by referring back to the original imaged volumetric data, which brings it back to the issue that the anatomical structures do usually not run parallel to an axis of the volume.

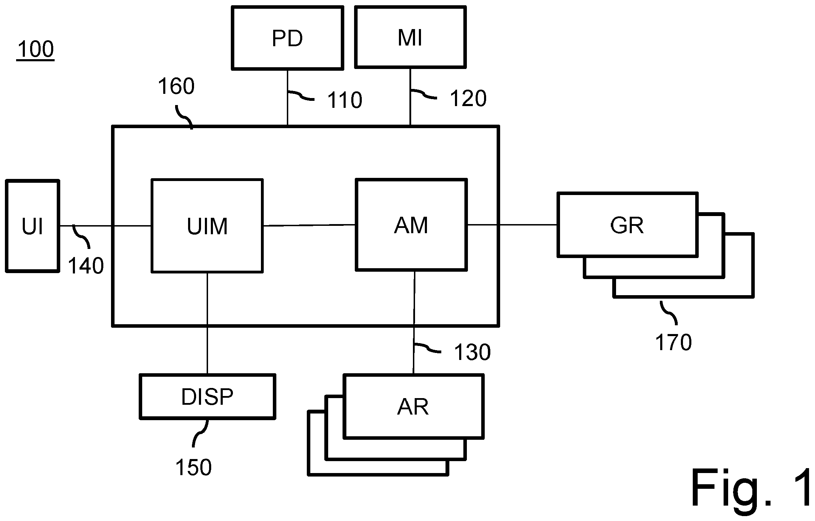

**Fig. 1** shows a system for processing medical image data regarding a clinical question. The system 100 has a patient data interface 110 for accessing patient data in a patient data repository, shown as a box PD, which contains patient information data and other diagnostic and treatment data. The system further has an image data interface 120 for accessing medical image data of a patient from a medical image data repository, shown as a box MI, which contains one or more medical imaging datasets of patients.

The system further has a repository interface 130 for accessing algorithms for processing the medical image data, shown as boxes AR. The algorithm repository AR may, for example, contain analytical algorithms, such as Artificial Intelligence (AI) algorithms for detection, characterization and segmentation of a tumor and surrounding anatomical structures. The algorithm repository AR may further contain visualization algorithms, which enable visualizations of the output data of the system.

The system further has a user interface 140 configured to receive user input from a user and to provide display data to a display 150 to visualize output data of the system. The user input may be received from a keyboard, mouse, joystick, touchscreen, voice interface, etc. The display may be a 2D display screen, but may also include other display units, e.g. a holographic 3D display or augmented reality (AR) glasses.

The system further has a processor subsystem 160 configured to communicate with the image data interface, the patient data interface, the repository interface and the user interface described above, and to perform the functions described below. Thereto, the processor subsystem may comprise a user interface manager UIM to manage user input and output, which UIM, for example, may include a layout, rendering and interaction manager. Also, the processor subsystem may comprise an algorithm manager AM, e.g. comprising an algorithm matching lookup table.

The processor subsystem is configured to determine a use case regarding the clinical question for a patient based on the user input. Subsequently, the patient data of the patient regarding the use case may be retrieved and an anatomical object is determined based on the use case and the patient data. Next, at least one algorithm is selected from the repository based on the determined anatomical object, for example an analytical algorithm to segment the anatomical object and/or a visualization algorithm to display the object. For example, a relevant AI model may be selected from the algorithm repository based on anatomical objects indicated in codified clinical guidelines. Further selection criteria may also be involved, as described below. Also, the image data of the patient is retrieved to be processed. Finally, the selected algorithm(s) are executed on the image data to provide the output data for assessing the clinical question.

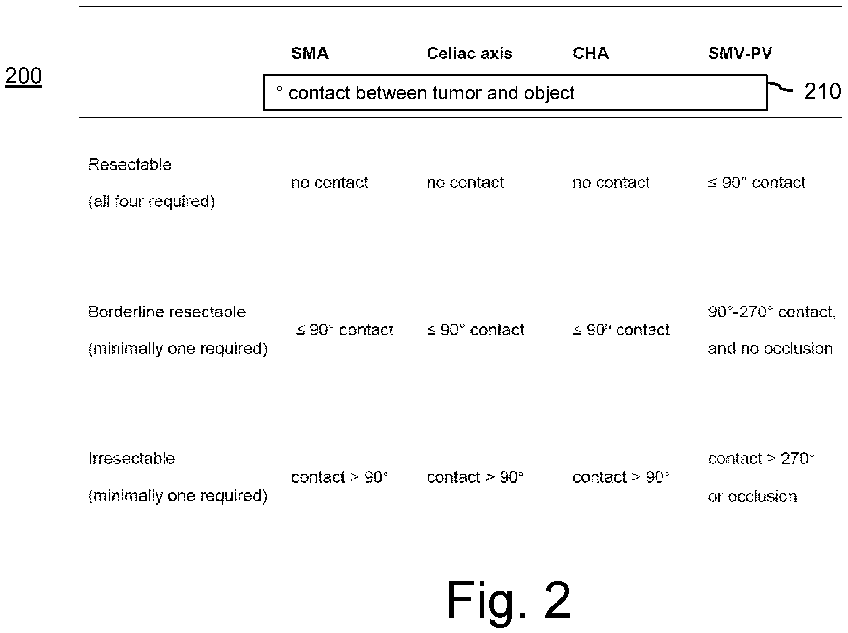

**Fig. 2** shows an example of codified guidelines. In the Fig. a table 200 shows a guideline with resectability criteria for pancreatic cancer (from: Dutch Pancreatic Cancer Group definitions for resectability of pancreatic adenocarcinoma, DPCG, 2012). The table has a first column that indicates guidelines, e.g. "Resectable, all four criteria required". The first row of the table shows anatomical objects or structures, such as blood vessels around an anatomical object which may be affected by a tumor, e.g. superior mesenteric artery (SMA), celiac axis, portal vein (PV), superior mesenteric vein SMV), common hepatic artery. In a field 210 metrics to be calculated are indicated, e.g. "contact between tumor and object". The second and further columns provide criteria for the guidelines as noted in the first column.

In an embodiment of the system described with Fig. 1 the processor subsystem 160 is configured to retrieve codified clinical guidelines 170 regarding the use case. Subsequently, one or more anatomical objects may be determined also based on the codified clinical guidelines. Also, algorithms may be selected also based on the codified clinical guidelines. The codified clinical guidelines may be stored in a guidelines repository (GR). The repository may contain integrated or self-defined guidelines or criteria for evaluation of anatomical structures and treatment decisions, for one or more disease types.

Optionally, the codified clinical guidelines for a use case define at least one of an anatomical object; metrics regarding relationships between anatomical objects; criteria regarding boundary values of the metrics; or guidance regarding clinical recommendations based on evaluation of the criteria. For example, guidelines may describe:
a. Objects; relevant anatomical objects (e.g. tumor and vascular structures such as the SMV-PV)
b. Metrics: relationship between objects (e.g. the degrees of contact between tumor and SMV-PV)
c. Criteria: clinically relevant cut-off values regarding the metrics (e.g. degrees of vascular involvement < 90° contact)
d. Guidance: the clinical recommendation based on evaluation of the criteria (e.g. resectable because of no contact with SMA, Celiac Axis and CHA, and <90° contact with SMV-PV).

Optionally, the processor subsystem has an algorithm matching lookup table for selecting the algorithm from the repository. The selection in the lookup table may be based on at least one of the determined anatomical object; the use case; the codified clinical guidelines; the metrics; the criteria; the guidance. The enhanced processing mechanism of the medical imaging viewing application or system is based on the algorithm matching look-up table for selecting an appropriate algorithm to be executed on the image data to assess the clinical question for the use case at hand.

Optionally, the processor subsystem is configured to select the algorithm from the repository containing analytic algorithms for calculating metrics, detection, characterization or segmentation of an anomaly, a tumor and/or an anatomical structure. The various analytical algorithms from the repository may generate, for example,
∘ Segmentations; e.g. of tumor, organ and vascular structures
∘ Classification scores; e.g. malignancy risk
∘ Recommendations: e.g. likelihood of resectability
∘ Derived metrics; e.g. degrees of vascular involvement (of vessel A, B, C), length of vascular involvement, extent of ingrowth.

Optionally, the processor subsystem is configured to select the algorithm from the repository containing visualization algorithms for visualization of the output data. The visualization algorithms may generate display data to show the output of the system while highlighting relevant clinical aspects, for example based on codified guidelines and available algorithm output, e.g. viewpoints in the 3D anatomical model, footprint representing contact area between segmented tumor and vessel.

Optionally, the processor subsystem comprises a layout rendering and interaction manager for selecting the visualization algorithm matching the output data as provided by the analytic algorithm for assessing the clinical question. The manager may render assessment panels and provide interaction options in line with the codified guidelines and available algorithm output.

The system may integrate codified guidelines containing evaluation criteria that describe relevant anatomical structures and metrics. It subsequently integrates a relevant set of algorithms that are applied to medical imaging data and provide output in line with these guidelines. The system may utilize the guidelines and algorithm output to drive the interface and interaction tools, with automatically configured layouts and viewpoints. The information may subsequently be displayed on 2D and 3D displays.

In practice, the system may be a software feature in existing image analysis and viewing software, e.g. provide modules in advanced visualization and image analysis software. Also, the system may be a software component that connects or augments currently separate software applications, e.g. bridges between the software marketplace and advanced visualization and image analysis software, and/or augment software with a hardware component and supporting software to enable a better connection to 3D display techniques.

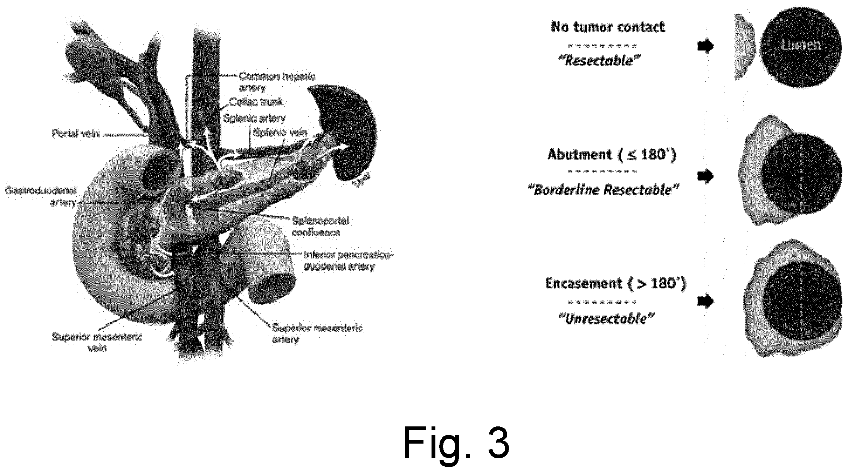

**Fig. 3** shows an example of a use case. The left part of the Fig. (from: https://radiologykey.com/) shows an anatomical organ, a pancreas, while the right part show treatments of the use case (from: Toesca et al. Int J Radiat Oncol Biol Phys. 2018 Apr 1;100(5): 1155-1174). For the use case, the pancreatic cancer guidelines as described with Fig. 2 may be applied. In the following embodiment the specific use case as shown in the Fig. is described.

Pancreatic cancer is an aggressive form of cancer with high mortality rate and extremely poor 5-year survival rates. The chances of survival are improved with early detection, swift diagnosis, thereby improving the chances of patients to receive surgery before the primary tumor has metastasized. Deciding if a patient is eligible for surgery is a complex decision involving multiple clinical stakeholders (e.g., radiologist, surgeon). A key driver in this decision is the degree of contact between the primary tumor and surrounding blood vessels: superior mesenteric artery (SMA), common hepatic artery (CHA), superior mesenteric vein (SMV) and portal vein (PV).

Assessment of contact is complex because relative positioning of these anatomical structures in a 3-dimensional space (i.e., the body) needs to be conducted based on CT scans on a per slice basis, followed by mental reconstruction of the CT scan into a mental 3D model. This leads to considerable variability in resectability assessment between medical specialists, resulting in suboptimal treatment selection: patients that are operable but are deemed inoperable do not get surgery or vice versa. Also, it is beneficial to the surgeon to have an optimal pre-surgical understanding of the patient-specific anatomy, so that they can define a patient-specific surgical plan even before starting the surgery. This surgical plan includes the navigation approach, preparation of required materials (e.g., vessel prostheses) and surgical staff (e.g., need for a vascular surgeon for complex vascular reconstruction).

The system as described above supports the clinical question in the above use case, i.e., resectability and surgical plan in the following ways.

The patient data repository 110 informs the system about the patient's diagnosis and treatment. This information may be present in structured format, e.g. through a Classification of Diseases (ICD) code describing the disease type and subcategory. In this embodiment, the diagnosis is pancreas cancer with possible surgical treatment. The patients relevant abdominal CT images are loaded into the system from the medical image data repository 120. Also, relevant accompanying clinical guidelines for pancreatic resectability may be loaded from the codified clinical guidelines repository 170.

Subsequently, the system identifies the object(s) relevant to this use case, e.g. the patient and guideline combination. In this embodiment it identifies five objects, namely the five relevant anatomical structures: tumor and surrounding blood vessels: superior mesenteric artery (SMA), common hepatic artery (CHA), superior mesenteric vein (SMV) and portal vein (PV) as shown in Fig. 3.

Next, the system identifies and calculates the metrics relevant to this use case. In this embodiment it identifies the metric relationship between objects, i.e. degrees of contact between tumor and the abovementioned vascular structures. Thereto, the system may call the algorithm matching look-up table to identify the relevant analytic algorithms required to determine the metrics. In this embodiment it identifies the segmentation algorithms of the tumor and surrounding blood vessels: superior mesenteric artery (SMA), common hepatic artery (CHA), superior mesenteric vein (SMV) and portal vein (PV) as the required algorithms. The system calls the relevant algorithms in the algorithm repository to run on the medical image data. The resulting anatomical segmentation data is used to calculate the metrics, e.g., degree of contact between tumor and SMV = 58 degrees.

Next, the metrics are matched to their corresponding criteria representing clinically relevant cut-off values regarding the metrics. In this embodiment, degrees of vascular involvement of the SMV = 58 < 90° contact with the tumor. This is repeated for other relevant criteria, e.g. superior mesenteric artery (SMA), common hepatic artery (CHA), superior mesenteric vein (SMV) and portal vein (PV). Guidance may be derived based on evaluation of the criteria as indicated in the codified guidelines, e.g. tumor is borderline resectable because of no contact with SMA, CHA, PV, and <90° contact with SMV

Next, the system calls the layout rendering and interaction manager to match the guidance with the corresponding layout, camera viewpoints and levels of transparency of anatomical structures most relevant to view the guidance as stored in the algorithm visualization repository.

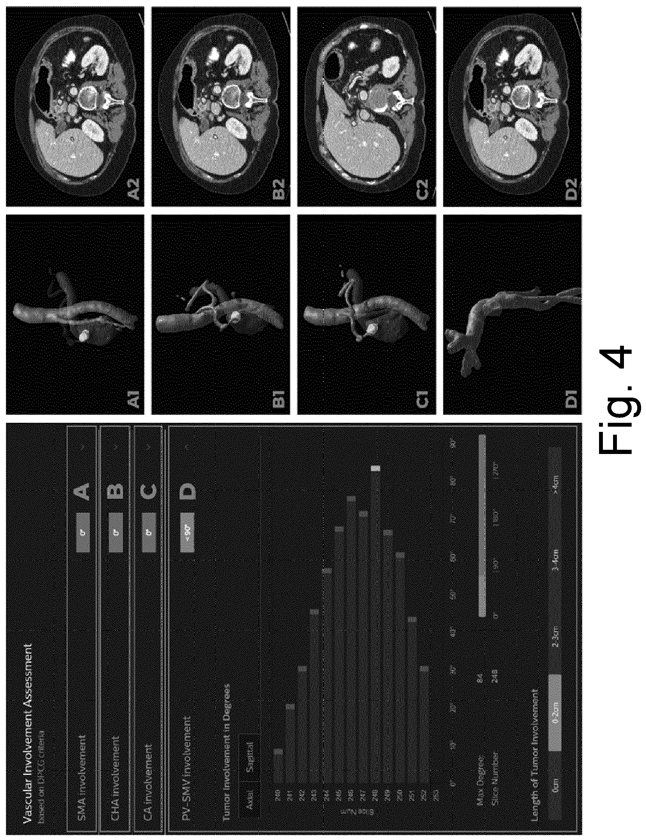

**Fig. 4** shows system output on a display. The system output uses different views, camera viewpoints and levels of transparency of relevant anatomical structures and is shown for the different decision criteria (A,B,C,D) as discussed above. For each criterium a 3D view is provided (panels A1, B1, C1, D1) and a respective slice in a 2D view (A2, B2, C2, D2). On the left side an interactive 2D panel is displayed showing metrics for criterium D for subsequent slices in bars having lengths proportional to the tumor involvement in degrees. The output may be displayed on a display unit, e.g. a large 2D screen, or a CT viewer and a 3D holographic display.

Optionally, the use case is determined as treatability based on the relation of a lesion with surrounding anatomical structures; or resectability of tumors in the vicinity of blood vessels. Furthermore, other use cases are described, e.g. other abdominal cancers such as liver cancer or colorectal liver metastases for which the suitability of therapy and therapy approach depends on involvement or vicinity of critical surrounding structures.

For abdominal cancers; e.g. liver cancer, an embodiment is described of stratification for and planning of liver cancer resection or locoregional therapy. Locoregional therapy options include ablation, arterially directed therapies (e.g. transarterial embolization) and radiotherapy. The feasibility of curative resection or suitability of locoregional therapies, depends on the following evaluation criteria:
- Performance status
- The Child-Pugh score (classification for the severity of liver disease)
- Adequate liver reserve
- Suitable liver remnant
- Radiologic tumor location and extent, e.g. Tumor location, Tumor size, Presence of single or multiple tumors, Presence of major vascular invasion (e.g. in portal vein, hepatic vein), Presence of lymph node or extrahepatic metastases
- Vicinity of major vascular structures and bile ducts

Codified guidelines may contain staging systems or treatment selection algorithms, of which a commonly used system, i.e. Barcolona Clinic Liver Cancer, BCLC is an example. The use case as defined by the patient data and the relevant guidelines is subsequently used for selecting the appropriate algorithms, as follows.

The system retrieves from the patient data repository the patient's diagnosis and treatment. In this use case, the diagnosis is liver cancer with possible surgical, ablative or arterially-directed treatment. The patient's relevant abdominal CT images are loaded into the system from the medical image data repository. The relevant clinical guidelines for liver cancer are loaded from the codified clinical guidelines repository. The system extracts clinical information, including the performance status (e.g. PS = 0), Child-Pugh score, liver reserve and liver remnant from the patient data repository. Besides, it extracts the cancer stage confirming absence of lymph node or extrahepatic metastases.

Subsequently, the system identifies the anatomical object(s) relevant to this use case. In this embodiment it identifies the following relevant anatomical structures: tumor, surrounding blood vessels and key anatomical structures; portal vein, hepatic vein, major bile ducts, and optionally nearby critical intra-abdominal structures and organs (e.g. gall bladder, pancreas, duodenum, stomach).

Also, the system identifies the metrics relevant to this use case. In this embodiment it identifies a metric relationship between objects identifiable on medical imaging: portal invasion by the tumor. The system uses the algorithm matching look-up table to identify the relevant analytical algorithms required to determine the metrics. In this embodiment it identifies segmentation algorithms of the tumor and surrounding blood vessels, i.e. the portal and hepatic arteries and veins. Optionally, it identifies segmentation algorithms for the specified intra-abdominal organs/structures. The system retrieves the relevant algorithms from the algorithm repository to run on the medical image data. The resulting anatomical segmentation data is used to calculate the metrics.

Next, the metrics may be matched to their corresponding criteria. In this embodiment, portal invasion is evaluated in binary fashion (absent/present). However, also a defined scale or score may be used. Guidance for a clinical decision is derived based on evaluation of the criteria, e.g. tumor is suitable for hepatic resection or locoregional therapy (ablation, or vascular directed therapy such as chemoembolization), because of absence of portal vein invasion.

Finally, the system engages the layout rendering and interaction manager to match the guidance with the corresponding layout, camera viewpoints and levels of transparency of anatomical structures most relevant to view the guidance as stored in the algorithm visualization repository. This generates different layouts, camera viewpoints and levels of transparency of anatomical structures. For further selection of the most appropriate procedure, besides tumor and portal vein, also the other critical surrounding organs may be displayed. Also said guidance may be shown on the display unit.

Optionally, patient-specific factors retrieved from the patient data may be part of the use case and may be used to influence guideline selection and evaluation criteria. For example, patient specific factors may mandate the use of other or more specific guidelines. An example is the surgical management of patients with liver cancer with portal vein tumor thrombosis (PVTT). Presence of such condition may influence which segmentations are required and which specific metrics need to be calculated. An example of a PVTT classification system is described in "Nevarez NM, Yopp AC. Challenging the Treatment Paradigm: Selecting Patients for Surgical Management of Hepatocellular Carcinoma with Portal Vein Tumor Thrombus. J Hepatocell Carcinoma. 2021;8:851-860". This guideline may lead to calculation of metrics related to tumor presence in 1st and 2nd order branching of the portal vein. In this case, on top of vascular segmentation alone, additional information is extracted as algorithm output; e.g. center lines of the vessels and coordinates of the branches, which is subsequently used to extract the part of the vessel affected by the thrombus and generate relevant viewpoints to inspect these parts of the vessels.

A further embodiment deals with abdominal cancers; e.g. colorectal liver metastases. As an example of criteria, here anatomical contra-indications for therapy using thermal ablation are based on: peritumoral vicinity (<10 mm) of the common, left or right hepatic bile duct, abutment of a single remaining major portal vein or hepatic vein and an unreconstructable invasion of the free wall of the inferior cava vein, as described in "Nieuwenhuizen et al. Resectability and Ablatability Criteria for the Treatment of Liver Only Colorectal Metastases: Multidisciplinary Consensus Document from the COLLISION Trial Group. Cancers (Basel). 2020 Jul 3;12(7):1779". The system provides integration of relevant algorithms to segment the tumor and these specified anatomical structures and renders the layouts and 3D viewpoints to evaluate these structures.

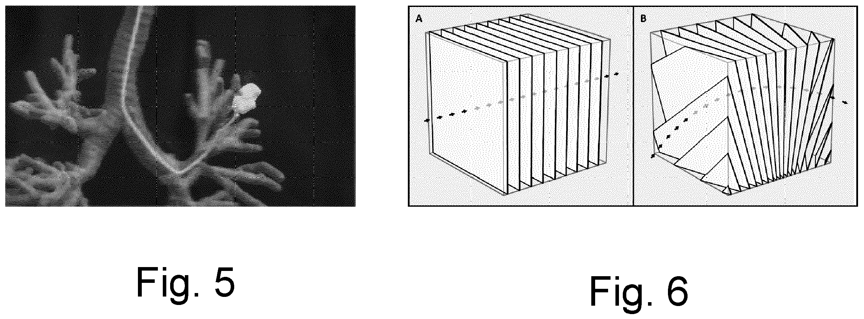

**Fig. 5** shows an illustrative example of lung biopsy planning with 3D guidance. The guidance is based on segmentation of the tumor, respiratory tree and simulated/planned biopsy path. The Fig. shows adjacent use of a visual guidance system for lung biopsy (and ablation) planning. In this embodiment, the visual guidance system can be used for planning of lung biopsy (and ablation) procedures. The use case is to improve biopsy accuracy and reduce time of biopsy procedures. For the biopsy procedure, the system integrates algorithms for segmentation of the lung tumor and bronchial tree and for prediction of the optimal biopsy path. The biopsy path is provided as a segmented structure as well as sequence of points along the biopsy path. Branching points of the bronchial tree may be derived from the segmentations. For visualization, the system calculates appropriate viewpoints in 3D, enabling to decide via which branching the bronchoscope should be introduced. These viewpoints keep in the field of view the position of the tumor (based on tumor coordinates) and the sequential branches along the navigation path (based on coordinates of the waypoints of the navigation path and branching points). Metrics can be determined related to complexity of the navigation at each branching point.

In a further embodiment, the processor subsystem comprises a natural language processing subsystem for determining the anatomical object and/or metrics related to the object based on the use case and the patient data. The natural language processing subsystem may be configured to process at least one of clinical guidelines regarding the use case, documents regarding the use case like radiology reports, or documents regarding the clinical question for identifying anatomical objects or structures, metrics, criteria or guidance relevant for the clinical question. The identification of relevant objects from the clinical guidelines is achieved using the natural language processing subsystem, also called Natural Language Processing (NLP) component. Relevant anatomical objects may be described with different terms and in combination with additional useful objects, like bile duct segmentation for pancreatic cancer identification, which are not explicitly mentioned in the clinical guidelines. The Natural Language component may be trained using the clinical guidelines, as well as research papers on the specific disease. In this manner the NLP component is able to identify the objects that are "semantically similar" and may complement the decision-making process of clinicians. During clinical use, the NLP component may identify relevant objects and may propose "semantically similar" objects to the clinicians to provide additional information. Similarly, relevant metrics may be found by the NLP component to leverage information from the research literature and to propose a list of relevant metrics that can assist in the clinical decision-making process.

Optionally, the processor subsystem is configured to determine the use case based on detecting an anomaly in the medical image data of an area of the body of the patient; and/or based on detecting an anatomical object or structure affected by an anomaly in the medical image data of an area of the body of the patient. The determined use case enables selecting said algorithm from the repository based on the detected anomaly and/or affected anatomical object or affected structure and retrieving the codified clinical guidelines.

In an embodiment, the image data comprises three-dimensional (3D) image data and the anatomical object is a 3D object in the 3D image data. The selected algorithm may be a slicing algorithm. In the embodiment, the processor subsystem is configured to determine an anatomically relevant path via the 3D object. Subsequently, the slicing algorithm is executed on the image data to provide, as the display data, slices of the 3D image data that are perpendicular to the anatomically relevant path for assessing the clinical question.

**Fig. 6** shows slicing of 3D volumetric data. In the Fig. the left part marked A shows a standard way following a straight path parallel to one of the volume's axes. The right part marked B shows slicing as proposed here, following a non-straight, anatomically relevant path that is relevant for the task at hand. Traditionally, as shown in part A, a slice does not provide an optimal cross section of an anatomical structure as the slicing is guided by the straight path of one of the axes of the volume. Instead using a non-straight path derived from a relevant anatomical structure to guide the slicing of the 3D imaging volume results in slices that provide an optimal cross section of an anatomical structure.

A practical clinical example is encountered in the assessment of the angle of the contact between a tumor and a blood vessel required to find out to what extent a tumor is resectable. If the angle is above a certain threshold, this means that there is too much contact between the tumor and blood vessel, making resection too risky.

The system is based on the above system having the following elements. The image data store 110 to store and retrieve 3D volumetric data, e.g., a picture archiving and communication system. The anatomically relevant path may be determined by an algorithm from the repository, e.g., a centre line of a blood vessel. A slicing algorithm may be used to compute a slice taken at an arbitrary angle from a 3D volume, for example using a 3×3 direction cosines matrix for the rotation and the 3D coordinates of centre of the rotation. A display is used to show a computed slice for assessment by a clinician.

Optionally, the processor subsystem (160) is configured to select a 2D algorithm from the repository for processing medical image data and to execute the 2D algorithm on a slice to calculate clinically relevant information. The selected 2D algorithm may apply 2D image processing to the slice to calculate clinically relevant information.

In an embodiment the slicing algorithm comprises the following steps. A first step is selecting a first position on the anatomically relevant path and extract first 3D coordinates of the first position. A next step is selecting a second position on the anatomically relevant path at a distance from the first position and extract second 3D coordinates of the second position. A next step is calculating a vector from the first position to the second position. A next step is computing a slice passing through the first position at an angle perpendicular to the vector. The steps may be repeated along the path.

In detail the processing may involve the following steps:
1. Select a position on the anatomically relevant path and extract the 3D coordinates of this position (i.e., the location of that spot within the 3D volume). This is the 'from' position.
2. Select a position on the anatomically relevant path that is at a small distance to the 'from' position and extract the 3D coordinates of this position. This is the 'to' position.
3. Calculate the normalized 'forward' vector originating in the 'from' position and pointing to the 'to' position.
4. From the 'forward' vector, calculate the normalized 'right' vector which points to the right-hand side of the 'from' position.
5. From the 'forward' vector and the 'right' vector, calculate the normalized 'up' vector which points upward from the 'from' position.
6. Construct a 3×3 direction cosines matrix from the 'forward', 'right', and 'up' vectors.
7. Provide the 3×3 direction cosines matrix and the coordinates of the 'from' position to the component/function that computes a slice of which its angle is determined by the direction cosines matrix and which passes through the 'from' position. The angle at which the slice is computed will be perpendicular to the 'forward' vector.
8. Send the computed slice to a system that can display it for assessment by a clinician or that can apply an algorithm to it to calculate clinically relevant information.
9. Select the current 'to' position as the new 'from' position.
10. If the end of the path is reached, stop here, else: go to step 2.

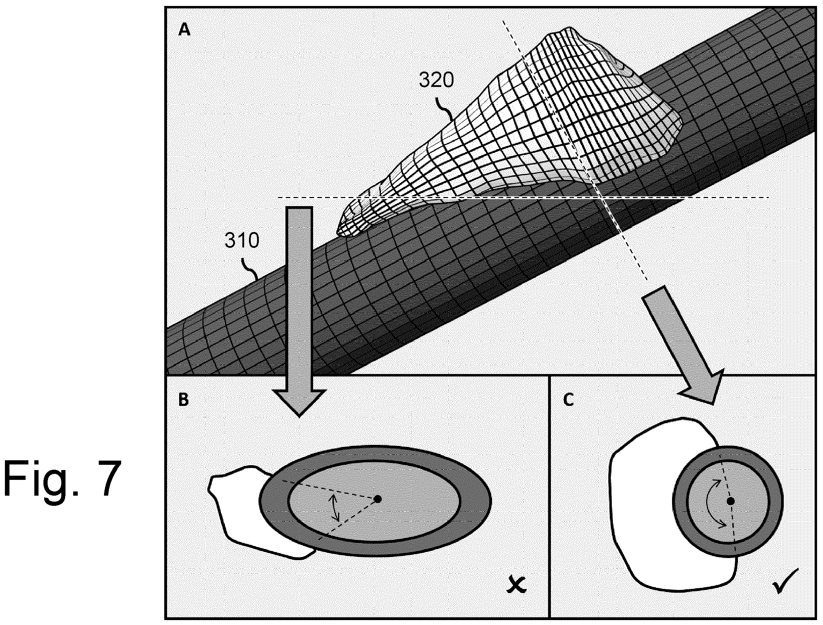

**Fig. 7** shows an example of a tumor that is in contact with an angled blood vessel. In the top part marked A, a blood vessel 310 is shown in contact with a tumor 320. Slicing directions are indicated by dashed lines, and resulting slices in left-bottom part B and in right-bottom part C.

Part A shows 3D volumetric data representing an angled blood vessel (dark grey) in contact with a tumor (white). The horizontal dashed line indicates how a standard axial slice would be taken in this situation. The angled dashed line is how a slice would be taken as proposed here when using the centre line of the blood vessel as the anatomically relevant path. Part B shows a slice obtained by standard axial slicing at the horizontal dashed lined. Because the slice was taken at a non-optimal angle as also apparent by the elongated shape of the blood vessel cross section, the contact angle of the tumor with the blood vessel is significantly underestimated. Part C shows a slice obtained by slicing as proposed here, perpendicular to the centre line of the blood vessel at the angled dashed line. In this case the slice is taken at the optimal angle as also clear from the rounded shape of the blood vessel cross section. The contact angle of the tumor with the blood vessel can now be correctly assessed.

If a 3D volume representing that situation would be sliced in the axial direction (along the z axis), the obtained slices would provide non-optimal cross sections of the situation, possibly leading to underestimation of the angles and therefore overestimation of the tumor's resectability. Such a traditional slice is shown in part B.

By using the centre line of the blood vessel as the path for slicing the volume, the slices will be perpendicular to the blood vessel and thereby provide optimal cross sections for assessment of the angles. Such an optimal, perpendicular slice is shown in part C.

In an embodiment, the anatomically relevant path is derived from the centre line of a blood vessel that is suspected to be in contact with a tumor. The computed centre line driven slices will then be perpendicular cross sections of the blood vessel along its centre line path. These slices can then be presented to a clinician in the same way as the standard orthogonal slices are presented (user can scroll through consecutive slices sequentially in a forward or backward direction). This will allow the clinician to assess possible tumor-vessel contact in an optimal way that is very intuitive and familiar, as the interaction is the same as with the standard views they already know.

In another embodiment, the anatomically relevant path is again derived from the centre line of a blood vessel that is suspected to be in contact with a tumor. In addition, segmentations (obtained from an algorithm or by manual annotation) for the blood vessel and the tumor are retrieved as 3D volumetric data. The centre line driven slicing will also be applied to these segmentations. This allows the sliced segmentations to be overlayed on the slices of the original 3D imaging volume for presentation to the clinician.

The sliced segmentations allow application of a robust 2D algorithm for the assessment of the angle of the tumor-vessel contact. Such an algorithm may, for example, have the following steps:
a. Set all tumor pixels where the blood vessel overlaps with the tumor to the background value (e.g., zero) to guarantee no overlap of tumor and blood vessel pixels.
b. Grow the shape of the tumor by 1 pixel by applying a morphological dilation step using a disk with a 1-pixel radius as the structuring element.
c. Apply a logical AND operation to the dilated tumor shape from step b and the blood vessel shape. This will yield the pixels on the blood vessel where the tumor is in contact with the blood vessel.
d. Retrieve the 2D coordinates of the pixels found in step c.
e. Retrieve the 2D coordinates of the blood vessel centre within the 2D slice based on the centre line of the blood vessel (note: these coordinates can also be found by calculating the centroid of the blood vessel pixels).
f. Calculate, using the 2-argument arctan (a.k.a. atan2), the angles between the coordinates of the blood vessel centre of step e and the coordinates of the contact pixels of step d.
g. Convert the angles of step f to a range of 0° to 360° by adding 360 to all negative angles.
h. Find the minimum angle from the angles of step g.
i. Find the maximum angle from the angles of step g.
j. Subtract the minimum angle of step h from the maximum angle of step i to get the angle of vascular involvement.
k. Calculate the mean of the angles of step g.
l. If the mean angle of step k is either less than the minimum angle of step h,
or more than the maximum angle of step i, it means that the tumor-vessel contact area starts at the contact pixel with the maximum angle and ends at the contact pixel with the minimum angle. In that case the angle of vascular involvement is the inverse of the angle found in step j. If so, subtract the angle of step j from 360 to get the correct angle of vascular involvement.

The angles of vascular involvement calculated by the algorithm for each slice can be presented to the clinician together with the computed centre line driven slices of the original imaged volume and the overlays of the sliced segmentations. This allows clinicians to easily verify if they agree with the found angles.

Optionally, the anatomically relevant path is first filtered to make the slice viewing experience smoother, as sometimes the change in direction along the unfiltered path can result in too large shifts in the looking direction of the slices.

Optionally, the number of points on the anatomically relevant path is made adaptive to the change in direction along the path, such that strong direction changes will lead to more points on the path and therefore the computed slices will be spaced closer together for a smoother viewing experience and to provide the maximum amount of data.

Optionally, the anatomically relevant path follows a route through the centre of lung bronchi all the way to a lung tumor at the end. The computed slices can help to pre-visualise the route for biopsy taking.

Optionally, the anatomically relevant path is derived from the perimeter of a tumor. The computed slices can provide more insight into the interface between the tumor and the surrounding tissue.

Optionally, the anatomically relevant path is derived from intestinal centre lines. The computed slices can offer unique insights into intestinal pathologies (especially in the intestinal lining) and can provide pre-visualisation to assist with surgical planning.

In another embodiment, the anatomically relevant path via the 3D object is determined along an interface of the 3D object and a further anatomical structure. A 3D volume is sliced following a path along the interface between the adjacent organs (e.g., a fat plane). The interface between the adjacent organs can then be inspected for anomalies.

Optionally, a coupling is made to a 3D reconstructed model of the volumetric data, such that the virtual camera is pointed in the same viewing direction as the anatomy-driven slice view, providing synced views of the original volumetric data and the 3D model. Also, a 3D ultrasound volume may be sliced using the centre line of a blood vessel to pre-visualise an intravascular ultrasound procedure.

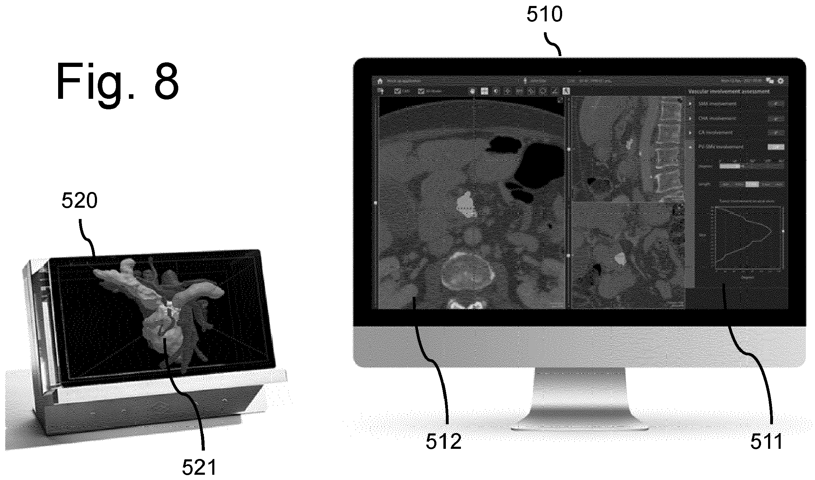

**Fig. 8** shows an example of the imaging system having a 2D display and a 3D display. The Fig. shows the system as described above in an integrated imaging workstation with a 2D display unit 510 and a 3D display unit 520. The elements of the system as described with Fig. 1 may be embedded in the workstation or display unit, or may be located in a separate enclosure (not shown). The 2D display unit may have a 2D view for displaying a guidance panel 511 showing the anatomical annotations, metrics, codified clinical guidelines and/or a slice 512 of the medical image data, and a 3D view for displaying the 3D anatomical model 521. The 3D view may also be presented on a 2D screen. The Fig. shows a true 3D display, e.g. using a 3D autostereoscopic display technique offering a true 3D perception to the user. Compared to a 3D model displayed on a 2D display, an improved depth perception is achieved. A 3D model on a 2D display may need turning the model to gain a depth understanding. The 2D views and the 3D view are integrated to operate as a single, synchronized working space. For example, when a 2D mouse cursor moves over the edge of a 2D view on the 2D display (image viewer and CAD outcome), it may automatically appear in to the 3D display and become a 3D cursor, and vice versa.

In a synchronizing embodiment, the selected algorithm is an analytical algorithm arranged to analyse the image data for generating a 3D anatomical model, anatomical annotations or metrics regarding the anatomical object. The processor subsystem is configured to synchronize the 3D view upon user input regarding the 2D view; or to synchronize the 2D view upon user input regarding the 3D view.

Synchronization may involve, in addition to taking a different 3D viewpoint for a virtual camera in the 3D view, adapting as well which information is presented or left out. Furthermore, a transitional effect may be included to move the camera position between the two viewpoints when a new view is selected. This may be accomplished by using animation motion tweening. Also the transparency values of segments can be animated, e.g. from a solid representation to translucent in the new view.

In the synchronizing embodiment the 2D view may be a metrics and quantifications guidance panel (e.g. vascular involvement assessment). The panel shows the guidelines (for example pancreas resectability criteria), with relevant calculated metrics the support oncological intervention planning. The 3D view may offer a 3D viewport for visualization and interaction with the 3D anatomical model. The 3D view could be provided on a standard 3D-on-2D viewer but also on an auto stereoscopic 3D holographic separate display.

In a further synchronizing embodiment, the processor subsystem is configured to synchronize a 3D viewpoint with respect to the 3D model upon user interaction in the 2D view comprising one of manipulating a cursor position in a slice; selecting anatomical annotations, metrics or codified clinical guidelines in the guidance panel; or to synchronize the 2D view upon change of a 3D cursor position in the 3D view by displaying a slice, anatomical annotations or metrics corresponding to the 3D cursor position.

The synchronizing embodiment synchronizes UI actions and visualizations in the 2D view and the 3D view. User interaction principles that help the clinician to perform a guidelines-based assessment. When synchronized, they integrate the behaviour of visualization of the standard image viewer, algorithm based assessments and a 3D model viewer of the reconstructed anatomical structures.

In practice synchronized views may be based on calculated metrics. For example, when selecting a vessel structure in the 2D guidance panel, the 3D view may automatically show the most optimal model to evaluate possible ingrowth of a tumor. Also, an interactive panel with calculated tumor involvement may be displayed per slice. When one of the structures is involved with the tumor, a panel opens to display per slice the calculated quantification. Only the relevant slices where there is involvement are shown together with metrics on the maximum angle and length of the involvement.

In Fig. 4 described above a display having a 2D guidance panel and a 3D view is presented. In addition a 2D slice view is presented. As mentioned above a metrics and quantifications step in the system generates for anatomical structures of interest a number of interesting metrics that are displayed in this interactive guidance panel. For assessment of vascular involvement, four vessels are relevant with respect to tumor involvement, as described above for the case of pancreatic cancer.

The synchronizing embodiment may synchronize 3D views based on calculated metrics presented in the 2D view. When clicking on a vessel structure in the 2D panel, the 3D view will show the most optimal model to a surgeon to evaluate the possible ingrowth.

For further evaluation, a surgeon may move the mouse cursor from the slice viewer or guidance panel to the 3D model view. In the 3D view, s/he can rotate and zoom into the model and change segment transparencies and switch segments on/off. Synchronized to this updated 3D model view, the slice viewer is controlled to show the most relevant slice. In the case of involvement the slice with maximum vascular involvement will be shown, in case of no vascular involvement the slice where the distance of the tumor to the vessel of interests is the smallest will be presented. Also the segment annotations may be overlayed on top of the slices, for example using a same colour coding as in the 3D model.

Optionally, the processor subsystem is configured to open an interactive display panel showing involvement of a tumor with the anatomical object and metrics regarding the involvement, or metrics regarding the involvement per slice; and to synchronize the interactive display panel, the 2D view and the 3D view. The embodiment may provide an interactive panel with calculated tumor involvement per slice. When one of the structures has involvement with the tumor, a panel opens displays per slice the calculated quantification. As shown by the bars in left panel in Fig. 4. So, a 'reverse' synchronization based on 3D interaction may also be provided. The surgeon may examine the 3D segmented model and click on a vessel structure. Now the guidance panel will open or adapt to show the involvement per slice. Also, relevant slices where there is involvement may be shown, optionally together with metrics on the maximum angle and length of the involvement.

The guidance panel may be interactive. When the surgeon clicks on one of the quantified vascular involvements, a crosshair in the 3D model, the slice viewer and the interactive vascular involvement chart may navigate based on the coordinate system to the point where the vascular involvement is detected. Also, the 'reverse' interaction is possible, while scrolling and moving a cursor or cross-hair in the slice view or positioning a 3D cursor on the model, other cursors or crosshairs will be aligned at the right slice in the vascular involvement interactive panel.

In an embodiment, the processor subsystem is configured to display the 3D anatomical model while at least one of the following 3D image processing acts is performed: removing non-relevant structures from the 3D model based on the use case; making the anatomical object (semi) transparent; choosing a viewpoint that shows a suitable angle to evaluate a tumor-vessel contact; making a footprint visible to clarify the area of contact between tumor and blood vessel and trajectory length of a tumor-vessel contact; adapting lighting settings for the 3D model visualization to highlight the tumor-vessel contact. Also, a potential change in material settings of the 3D model (segments), or a different shader (visual) effect may be applied, or a change of virtual camera settings like focus depth, or additional texture and the like may be added. In practice, one or more of the following steps are performed:
a. All non-relevant structures are removed from the 3D model. For example, a vessel that is further away and has no connection or intervening with the tumor is removed,
b. The pancreas is made transparent or partially transparent, to have a clear view on the tumor and vessel structure that is selected,
c. a viewpoint is chosen, that shows the most optimal angle to evaluate the tumor-vessel contact,
d. the footprint can be made visible to clarify tumor-vessel contact area and trajectory length of tumor-vessel contact,
e. possibly the lighting settings or other rendering settings for the 3D model visualization are adapted to highlight the tumor-vessel contact. These settings may include for example: light position, direction and intensity, and may include multiple light sources.

Optionally, a tumor footprint in the 3D model may be determined. An additional non-anatomical structural annotation may be calculated and used to get a better 3D understanding of the vascular tumor involvement. This annotation is called the tumor footprint, which is defined as the 2D projection of contact area of tumor on the vessel. Also, additional information of the innerworkings of the selected algorithm could be added on top of the footprint and the tumor, such as a heatmap. This will give a clinician more insight in the certainty of the tumor prediction of the algorithm.

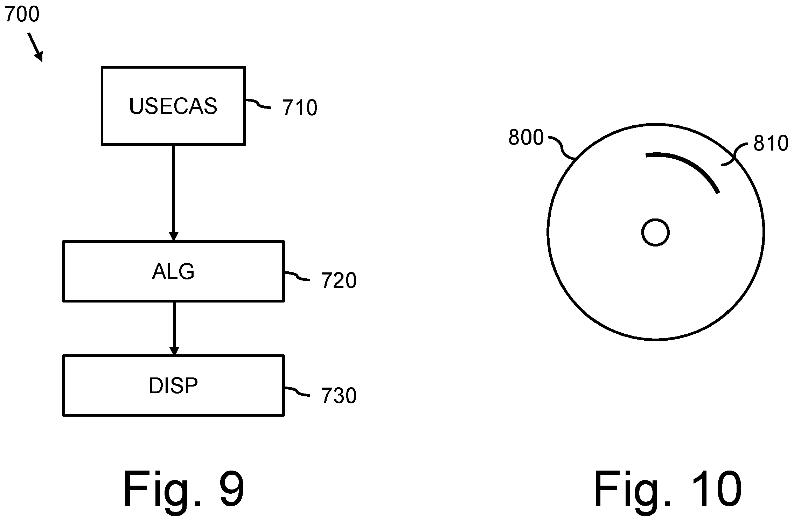

**Fig. 9** shows a flow-diagram of a computer-implemented method 700 for processing medical image data regarding a clinical question. The method 700 may correspond to an operation of the system 100 of Fig. 1. The processing comprises accessing patient data; accessing medical image data of a patient; accessing algorithms for processing the medical image data; receiving user input from a user; providing display data to visualize output data of the system.

The method 700 may comprise, in an operation titled "USECAS" 710, determining a use case regarding the clinical question for a patient based on the user input and retrieving the patient data of the patient regarding the use case, and determining an anatomical object based on the use case and the patient data. A further operation titled "ALG" 720 involves selecting an algorithm based on the determined anatomical object. A further operation titled "DISP" 730 includes retrieving the image data of the patient and executing the algorithm on the image data to provide the output data for assessing the clinical question.

It will be appreciated that, in general, the operations of method 700 of Fig. 8 may be performed in any suitable order, e.g., consecutively, repeatedly, simultaneously, or a combination thereof, subject to, where applicable, a particular order being necessitated, e.g., by input/output relations.

The method(s) may be implemented on a computer as a computer implemented method, as dedicated hardware, or as a combination of both. As illustrated in **Fig. 10**, instructions for the computer, e.g., executable code, may be stored on a computer readable medium 800, e.g., in the form of a series 810 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 10 shows an optical disc 800. Alternatively, the computer readable medium 800 may comprise transitory or non-transitory data 810 representing the machine trained model as described elsewhere in this specification.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for processing medical image data regarding a clinical question, comprising:
- a patient data interface (110) for accessing patient data;
- an image data interface (120) for accessing medical image data of a patient;
- a repository interface (130) for accessing algorithms for processing the medical image data;
- a user interface (140) configured to receive user input from a user and to provide display data to a display (150) to visualize output data of the system;
- a processor subsystem (160) configured:
to communicate with the image data interface, the patient data interface, the repository interface and the user interface;
to determine a use case regarding the clinical question for a patient based on the user input;
to retrieve the patient data of the patient regarding the use case;
to determine an anatomical object based on the use case and the patient data;
to select an algorithm from the repository based on the determined anatomical object;
to retrieve the image data of the patient; and
to execute the algorithm on the image data to provide the output data for assessing the clinical question.

2. The system (100) according to claim 1, wherein the processor subsystem (160) is configured:
to retrieve codified clinical guidelines (170) regarding the use case; and
to determine said anatomical object, and to select said algorithm, also based on the codified clinical guidelines.

3. The system (100) according to claim 2, wherein the codified clinical guidelines for a use case define at least one of:
anatomical object;
metrics regarding relationships between anatomical objects;
criteria regarding boundary values of the metrics;
guidance regarding clinical recommendations based on evaluation of the criteria.

4. The system (100) according to claim 1, 2 or 3, wherein the processor subsystem (160) comprises an algorithm matching lookup table for selecting the algorithm from the repository based on at least one of
the determined anatomical object;
the use case;
the codified clinical guidelines;
the metrics;
the criteria;
the guidance.

5. The system (100) according to any of the preceding claims, wherein the processor subsystem (160) is configured to select the algorithm from the repository containing analytic algorithms for calculating metrics, detection, characterization or segmentation of an anomaly, a tumor and/or an anatomical structure.

6. The system (100) according to any of the preceding claims, wherein the processor subsystem (160) is configured to select the algorithm from the repository containing visualization algorithms for visualization of the output data.

7. The system (100) according to claim 5 and 6, wherein the processor subsystem (160) comprises a layout rendering and interaction manager for selecting the visualization algorithm matching the output data as provided by the analytic algorithm for assessing the clinical question.

8. The system (100) according to any of the preceding claims, wherein the processor subsystem (160) comprises a natural language processing subsystem for determining the anatomical object and/or metrics related to the object based on the use case and the patient data, wherein the natural language processing subsystem is configured to process at least one of:
clinical guidelines regarding the use case;
documents regarding the use case;
documents regarding the clinical question;
for identifying anatomical objects or structures, metrics, criteria or guidance relevant for the clinical question.

9. The system (100) according to any of the preceding claims, wherein the processor subsystem (160) is configured to determine the use case:
based on detecting an anomaly in the medical image data of an area of the body of the patient; and/or
based on detecting an anatomical object or structure affected by an anomaly in the medical image data of an area of the body of the patient;
for enabling selecting said algorithm from the repository based on the detected anomaly and/or affected anatomical object or affected structure and retrieving the codified clinical guidelines.

10. The system (100) according to any of the preceding claims, wherein the use case is
treatability based on the relation of a lesion with surrounding anatomical structures; or
resectability of tumors in the vicinity of blood vessels.

11. The system (100) according to any of the preceding claims, wherein the image data comprises three-dimensional (3D) image data;
the anatomical object is a 3D object in the 3D image data;
the selected algorithm is a slicing algorithm; and
the processor subsystem (160) is configured:
to determine an anatomically relevant path via the 3D object;
to execute the slicing algorithm on the image data to provide, as the display data, slices of the 3D image data that are perpendicular to the anatomically relevant path for assessing the clinical question.

12. The system (100) according to claim 11, wherein the slicing algorithm comprises the steps of:
selecting a first position on the anatomically relevant path and extract first 3D coordinates of the first position;
selecting a second position on the anatomically relevant path at a distance from the first position and extract second 3D coordinates of the second position;
calculating a vector from the first position to the second position;
computing a slice passing through the first position at an angle perpendicular to the vector.

13. The system (100) according to any of the claims 11 or 12, wherein the processor subsystem (160) is configured:
to select a 2D algorithm from the repository for processing medical image data;
to execute the 2D algorithm on a slice to calculate clinically relevant information.

14. The system (100) according to any of the claims 11 to 13, wherein the anatomically relevant path via the 3D object is determined along an interface of the 3D object and a further anatomical structure.

15. The system (100) according to any of the preceding claims, wherein
the selected algorithm is an analytical algorithm arranged to analyse the image data for generating a 3D anatomical model, anatomical annotations or metrics regarding the anatomical object;
the display comprises
a 2D view for displaying a guidance panel showing the anatomical annotations, metrics, codified clinical guidelines and/or a slice of the medical image data, and
a 3D view for displaying the 3D anatomical model;
the processor subsystem (160) is configured to synchronize the 3D view upon user input regarding the 2D view; or to synchronize the 2D view upon user input regarding the 3D view.

16. The system (100) according to claim 15, wherein the processor subsystem (160) is configured
to synchronize a 3D viewpoint with respect to the 3D model upon user interaction in the 2D view comprising one of
manipulating a cursor position in a slice;
selecting anatomical annotations, metrics or codified clinical guidelines in the guidance panel; or
to synchronize the 2D view upon change of a 3D cursor position in the 3D view by displaying a slice, anatomical annotations or metrics corresponding to the 3D cursor position.

17. The system (100) according to claim 15 or 16, wherein the processor subsystem (160) is configured:
to open an interactive display panel showing involvement of a tumor with the anatomical object and metrics regarding the involvement, or metrics regarding the involvement per slice; and
to synchronize the interactive display panel, the 2D view and the 3D view.

18. The system (100) according to any of the claims 15 to 17, wherein
the processor subsystem (160) is configured to display the 3D anatomical model while at least one of:
removing non-relevant structures from the 3D model based on the use case;
making the anatomical object transparent;
choosing a viewpoint that shows a suitable angle to evaluate a tumor-vessel contact;
making a footprint visible to clarify the area of contact and trajectory length of a tumor-vessel contact;
adapting lighting settings for the 3D model visualization to highlight the tumor-vessel contact.

19. The system (100) according to any of the preceding claims, the system comprising the display (150) having
a 2D display for displaying a guidance panel showing the anatomical annotations, metrics, codified clinical guidelines and/or a slice of the medical image data, and
a 3D display for displaying a 3D anatomical model of the anatomical object.

20. A computer-implemented method (700) for processing medical image data regarding a clinical question, the processing comprising:
- accessing patient data;
- accessing medical image data of a patient;
- accessing algorithms for processing the medical image data;
- receiving user input from a user;
- providing display data to visualize output data of the system;
wherein the method comprises the steps of
determining a use case regarding the clinical question for a patient based on the user input;
retrieving the patient data of the patient regarding the use case;
determining an anatomical object based on the use case and the patient data;
selecting an algorithm based on the determined anatomical object;
retrieving the image data of the patient; and
executing the algorithm on the image data to provide the output data for assessing the clinical question.

21. A computer-readable medium (800) comprising transitory or non-transitory data (810) representing a computer program, the computer program comprising instructions for causing a processor system to perform the method according to claim 20.
